# EUROPEAN PATENT APPLICATION

(11) **EP 2 127 639 A1**
(43) Date of publication of application: **02.12.2009**
(21) Application number: 08156887.5
(22) Date of filing: 26.05.2008
(51) Int. Cl.: A61K 9/127, A61K 31/573, A61P 9/10

(54) **Corticosteroid containing liposomes for treatment of cardiovascular diseases**

(71) Applicant: Universiteit Utrecht Holding B.V., 3584 CM Utrecht (NL); Mount Sinai School of Medicine of New York University, New York, NY 10029 (US)
(72) Inventor: Mulder, Willem Jan Menno, New York, NY 10029 (US); Fayad, Zahi Adel, New York, NY 10538 (US); Storm, Gerrit, 3813 CN Amersfoort (NL); Metselaar, Josbert Maarten, 1094 ND Amsterdam (NL)
(74) Representative: Lahrtz, Fritz

(57) **Abstract**

The invention provides a use of a long-circulating microvesicle comprising a sterol, partially synthetic or wholly synthetic vesicle-forming phospholipids, and a corticosteroid in water soluble form, which microvesicle has a mean particle diameter size range of between about 75 and 150 nm and which microvesicle is non-charged or negatively charged at physiological conditions, for the preparation of a medicament for the treatment of atherosclerosis and/or cardiovascular disease. A method for treating a subject suffering from, or at risk of suffering from, atherosclerosis and/or cardiovascular disease, comprising administering to said subject a therapeutically effective amount of such long-circulating microvesicles is also provided.

## Description

The invention relates to the fields of biology and medicine.

Atherosclerosis is a leading cause of illness and death in most Western countries. It can affect the heart, brain, other vital organs, and/or extremities. Atherosclerosis involves deposits of fatty substances, cholesterol, cellular waste products, calcium, and/or fibrin in the inner lining of an artery. Atherosclerosis is an inflammatory disease. One of the main factors contributing to the buildup of this disease is macrophage accumulation into the arterial vessel wall. Macrophage infiltration has been identified as a key event in the progression of this disease that may ultimately result in clinical events such as stroke and myocardial infarction. Cardiovascular morbidity and mortality related to atherosclerosis affects almost 1 million in the US alone, while the yearly costs related to treatment of atherosclerosis are estimated to exceed 360 billion US dollars. One of the approaches that have been proposed to tackle this disease is resolving/treating the chronic inflammation associated to the disease, which has been identified to occur and build up in patients as young as teenagers.
One of the drug classes that has been studied in the treatment of inflammation in developing atherosclerotic lesions are glucocorticoids. An early study with dexamethasone in cholesterol fed rabbits clearly demonstrated the inhibitory effect on macrophage accumulation in the intima and media of an atherosclerotic lesion (Poon M, Gertz SD, Fallon JT, Wiegman P, Berman JW, Sarembock IJ, Taubman MB. Dexamethasone inhibits macrophage accumulation after balloon arterial injury in cholesterol fed rabbits. Atherosclerosis. 2001 Apr;155(2):371-80).
Recently, Ribichini *et al*. (Ribichini F, Joner M, Ferrero V, Finn AV, Crimins J, Nakazawa G, Acampado E, Kolodgie FD, Vassanelli C, Virmani R. Effects of oral prednisone after stenting in a rabbit model of established atherosclerosis. J Am Coll Cardiol. 2007 Jul 10;50(2):176-85. Epub 2007 Jun 22.) have shown reduced neo-intimal formation in prednisone- and Taxus stent-treated animals by suppressing several inflammatory pathways after interacting with NF-kappa B. The reduced transcription of various proinflammatory genes resulted in diminished release of inflammatory cytokines, chemokines, and cell adhesion molecules. Glucocorticoids are powerful anti-inflammatory agents that suppress many phlogistic responses including inflammatory cell recruitment and activation (Goulding NJ, Guyre PM. Glucocorticoids, lipocortins and the immune response. Curr Opin Immunol. 1993 Feb;5(1):108-13) and have the ability to induce apoptosis at high concentrations. They have also been proposed to stimulate phagocytic clearance of apoptotic cells (Maderna P, Godson C. Phagocytosis of apoptotic cells and the resolution of inflammation. Biochim Biophys Acta. 2003 Nov 20;1639(3):141-51), which should facilitate the inhibition of inflammation. As of yet, they have not been applied for treatment of atherosclerosis in the clinic due to a variety of reasons.

First, for effective treatment of diseases that are not easily accessible and can only be accessed from the circulation, as is the case with atherosclerosis, intravenous administration is indicated. Free circulating glucocorticoids have a very low circulatory half-life and poor pharmacokinetics, causing low drug concentrations at sites of desired action, rendering them ineffective in treatment which requires high dosages and frequent administration. In addition to having a short circulation half-life, free circulating glucocorticoids cause an array of adverse systemic effects, including *e*.*g*. deregulation of physiological corticosteroid levels, osteoporosis, hypertension and myopathy. These undesired side-effects may cause possible risks and potentially outweigh benefits of treatment, making free circulating glucocorticoids not suitable for treatment of atherosclerosis in patients.

As an alternative to free circulating glucocorticoids, it has been proposed to incorporate dexamethasone palmitate into lipid microspheres (Chono et al, 2005a). These microspheres were reported to have an anti-atherosclerotic effect in atherogenic mice. However, these microspheres have a short half-life (they were rapidly cleared from the circulation), so that the use of these microspheres involves the above mentioned disadvantages of low drug concentrations at sites of desired action which requires high dosages and frequent administration. It has also been proposed to use dexamethasone which has been incorporated into liposomes composed of egg yolk phosphatidylcholine, cholesterol and dicetylphosphate (Chono et al, 2005b). Although these liposomes exhibit anti-atherosclerotic effects, they have a short half-life of less than 6 hours, which involves the above mentioned disadvantage of low drug concentrations at sites of desired action. Particles with a short half-life are not efficient for human applications, because this would require high dosages and frequent administration.

Hence, there is an ongoing need for alternative therapies and therapeutic compositions.

It is an object of the present invention to provide alternative means, methods and compositions for counteracting and/or at least in part preventing atherosclerosis and/or a cardiovascular disease. It is a further object to provide compositions for counteracting and/or at least in part preventing atherosclerosis and/or a cardiovascular disease, which compositions have improved pharmacokinetics as compared to conventional anti-atherosclerotic drugs. It is a further object to provide compositions for counteracting and/or at least in part preventing atherosclerosis and/or a cardiovascular disease, which compositions provide higher drug concentrations at sites of desired action, allowing lower administration dosages and/or resulting in less adverse systemic effects, as compared to conventional anti-atherosclerotic drugs. The present invention is also directed to a method to use microvesicles to encapsulate corticosteroids and use of these systems for delivery at a site of atheroslerosis. It is a further object to provide means, methods and compositions for delivery of therapeutics to a site where plaque is present.

In accordance with the present invention, it has now been found that compositions comprising a corticosteroid in water soluble form, encapsulated in particular types of long-circulating microvesicles, are particularly suitable for counteracting and/or at least in part preventing atherosclerosis and/or cardiovascular disease. These compositions are thus particularly suitable for the preparation of a medicament against these diseases.

Accordingly, the present invention provides a use of a long-circulating microvesicle comprising a sterol, partially synthetic or wholly synthetic vesicle-forming phospholipids, and a corticosteroid in water soluble form, which microvesicle has a mean particle diameter size range of between about 75 and 150 nm and which microvesicle is non-charged or negatively charged at physiological conditions, for the preparation of a medicament for the treatment of atherosclerosis and/or cardiovascular disease and/or plaque.

The present invention relates to the use of a corticosteroid in water soluble form encapsulated in a long-circulating microvesicle for the manufacture of a medicament useful in counteracting, treating and/or at least in part preventing atherosclerosis and/or cardiovascular disease and/or plaque. Compositions according to the invention were found to deliver a corticosteroid at a site of atherosclerosis and can hence be used for efficient treatment of atherosclerosis and/or cardiovascular disease and/or plaque.

The long-circulating microvesicles have very favourable pharmacokinetics, a favourable tissue distribution behaviour and an efficient half-life. Additionally, a stable association between corticosteroid and the carrier system, the microvesicles, is observed, while the loading with corticosteroid is efficient. Further a good biological availability at a site of atherosclerosis where activity is required is observed. Without wishing to be bound by any theory, it is hypothesized that the microvesicles have an interaction with macrophages which are present at a site of atherosclerosis. Even though it would be expected that the interaction between long-circulating microvesicles and macrophages would be low (allowing the microvesicles to remain in the circulation during a prolonged period of time), it has been found by the present invention that long-circulating microvesicles are nevertheless particularly suitable for counteracting atherosclerosis and/or cardiovascular disease because they are capable of efficiently delivering a corticosteroid at a site of atherosclerosis. The microvesicles according to the present invention are capable of accumulating at a site of atherosclerosis, thereby minimizing systemic exposure of corticosteroid and thus avoiding or diminishing adverse systemic effects of corticosteroids, including *e*.*g*. deregulation of physiological corticosteroid levels, osteoporosis, hypertension and myopathy.

In a preferred embodiment a long-circulating microvesicle according to the invention is a liposome, a nanocapsule or a polymeric micelle. A use according to the invention, wherein said microvesicle is selected from the group consisting of liposomes, nanocapsules and polymeric micelles, is therefore also provided.

Other suitable long-circulating microvesicles can be based on lipoproteins, and especially high density lipoproteins and low density lipoproteins, and on lipoprotein mimetics or neo-lipoproteins.

It has been found that long-circulating microvesicles, and especially long-circulating liposomes, nanocapsules and polymeric micelles, are capable of efficiently delivering corticosteroids drugs to atherosclerotic plaques. In particular, the present invention provides a medicament for or in the treatment of atherosclerosis and/or cardiovascular disease, suitable to administer corticosteroids, and especially glucocorticoids, in relatively low dosages. In accordance with the invention, effective inhibition of inflammation in atherosclerosis has been observed in particular embodiments with relatively low dosages of only 15 mg/kg body weight per week.

Without wishing to be bound by any theory, it is believed that microvesicles used in the present invention accumulate at sites of atherosclerosis as a result of the enhanced permeability of atherosclerotic vasculature as compared to healthy endothelium, allowing an improved localization and improved retention of the corticosteroid at these sites.

The long-circulating microvesicles used in accordance with the present invention typically have a mean particle diameter of about 75-150 nm, as determined by Dynamic light scattering using a Malvern 4700 ™ system equipped with a He/Ne laser. The microvesicles of the invention preferably have a rather small polydispersity which means that the particle size distribution is narrow. Preferably, the polydispersity index, which is calculated by the software belonging to the dynamic light scattering equipment, is less than 0.25, and more preferably less than 0.2.

Microvesicles according to the present invention comprise a sterol, partially synthetic or wholly synthetic vesicle-forming phospholipids and a corticosteroid in water soluble form. Sterols are well known in the art. Sterols, or steroid alcohols are a subgroup of steroids with a hydroxyl group in the 3-position of the A-ring. They are amphipathic lipids synthetised from acetyl-coenzyme A. The hydroxyl group on the A ring is polar. The rest of the aliphatic chain is non-polar.

Cholesterol is one of the most important sterols. The use of cholesterol is preferred because, using cholesterol, particular stable microvesicles with a long half-life are obtained. For instance, it has been demonstrated that cholesterol-containing microvesicles according to the invention have a half-life of > 24 h in rabbits. Hence, in a preferred embodiment a use of a long-circulating microvesicle according to the invention is provided, wherein said sterol comprises cholesterol.

As used herein, the term "partially synthetic or wholly synthetic vesicle-forming phospholipids" means at least one vesicle-forming phospholipid which has either been artificially made or which originates from a naturally occurring phospholipid, which has been artificially modified. The use of partially synthetic or wholly synthetic vesicle-forming phospholipids increases the stability and, hence, the half-life of microvesicles according to the present invention. Preferred phospholipids contain saturated alkyl chains, yielding a bilayer with a relatively high transition temperature. Particularly preferred phospholipids are distearoyl phosphatidylcholine (DSPC), dipalmitoyl phosphatidylcholine (DPPC), Hydrogenated Soya Phosphatidyl Choline (HSPC) and hexadecylphosphocholine (HEPC). Microvesicles according to the invention comprising DSPC, DPPC, HSPC and HEPC are particularly stable and have a long half-life of at least three hours. Further provided is therefore a use according to the invention, wherein said partially synthetic or wholly synthetic vesicle-forming phospholipids are selected from the group consisting of DSPC, DPPC, HSPC and HEPC.

The microvesicles used in compositions according to the invention are long-circulating liposomes. As used herein, the term "long-circulating microvesicle" means a microvesicle which has a half-life of at least 3 hours. Preferably, a long-circulating microvesicle according to the invention is used which has a half-life of at least 6 hours. The term "half-life" is defined herein as the time where after half of the amount of microvesicles administered to an animal has been degraded. It is preferably expressed as the time at which the second linear phase of the logarithmic microvesicle clearance profile reaches 50% of its initial concentration, which initial concentration is the extrapolated plasma concentration at t=0.

Using long-circulating microvesicles according to the present invention, it has been found that atherosclerotic plaque inflammation and neovascularization is reduced with more than 30% and even op to 70% compared to controls, at a dose of only 15 mg microvesicles per kg body weight per week.

Long-circulating liposomes are already known in the art, even in combination with corticosteroids. More particularly, known liposome systems are described in WO 03/105805 and WO 02/45688. For the preparation of suitable compositions to be used in the present invention, the preparation methods described in WO 03/105805 and WO 02/45688 are incorporated herein by reference. The use of long-circulating microvesicles according to the present invention for counteracting and/or at least in part preventing atherosclerosis and/or cardiovascular disease is not disclosed nor suggested in these patent applications.

Microvesicles according to the present invention are non-charged or negatively charged at physiological conditions. This means that the overall charge of the microvesicles is neutral or negatively charged at a physiological pH of between 6 and 8. This provides the advantage, as compared to positively charged microvesicles, that the time that the microvesicles according to the present invention remain in the human/animal circulation is significantly increased. Hence, the circulation half life is prolonged as compared to positively charged microvesicles. The microvesicles according to the present invention are less quickly eliminated by an individual's immune system because they lack positive charge. Therefore, the targeting potential of the microvesicles according to the present invention is also improved, meaning that an increased localisation and improved retention of the microvesicles at a site of interest such as atherosclerotic tissue is obtained.

In a particularly preferred embodiment, microvesicles according to the present invention are used which comprise at most 10 mole percent of negatively charged vesicle-forming phospholipids, based upon the molar ratio of the vesicle forming lipids. Preferably, between 5-10 mole % of negatively charged phospholipids are present. Negatively-charged microvesicles according to the invention are particularly stable, so that the time that these negatively-charged microvesicles remain in the human/animal circulation is significantly increased.

Non-limiting examples of suitable charged vesicle-forming lipids are phosphatidylglycerol, phosphatidylethanolamine, (di)stearylamine, phosphatidylserine, dioleoyl trimethylammonium propane, phosphatidic acids and cholesterol hemisuccinate.

Where in this description reference is made to charged/uncharged/amphiphatic, and so on, this reference relates to physiological conditions.

A microvesicle according to the invention preferably comprises at least one type of polymer lipid conjugates, such as lipids derivatised with polyalkylene glycol, preferably with polyethylene glycol (PEG). Yet another preferred embodiment thus provides a use of microvesicles according to the present invention, which microvesicles further comprise polyethylene glycol (PEG). The incorporation of PEG further increases the stability of the microvesicles.

Suitable polymer-lipid-conjugates have a molecular weight of between 200 and 30,000 Dalton. Other suitable candidates to be used in these polymer-lipid-conjugates or water-soluble polymers such as: poly ((derivatized) carbohydrate)s, water-soluble vinylpolymers (*e*.*g*. poly(vinylpyrrolidone), polyacrylamide and poly(acryloylmorpholine) and poly(methyl/ethyl oxazone). These polymers are coupled to the lipid through conventional anchoring molecules. Suitably, the concentration of polymer lipid conjugates is 0-20 mole%, and preferably 1-10 mole%, based upon the total molar ratio of the vesicle forming lipids.

The presence of these polymer-lipid-conjugates has a favourable effect on the circulation time. However, by carefully selecting specific lipid compositions at physical specifications, suitable long circulation times can be obtained without using a polymer-lipid-conjugate. For example, 50-100 nm liposomes of distearylphopshatidylcholine and cholesterol and/or sphingolipids like sphingomyelin are suitable.

In a particularly preferred embodiment, the invention provides a use of a microvesicle according to the invention, wherein said microvesicle is a liposome comprising 0-50 mol% of cholesterol, 50-90 mol% of non-charged partially synthetic or wholly synthetic vesicle-forming lipids, 0-20 mol% of amphipatic vesicle-forming lipids coupled to polyethylene glycol, and 0-20 mol% of a negatively charged vesicle-forming lipid. Such liposome is for instance made in accordance with the methods described in WO 02/45688. The liposomes have a mean particle diameter size range of between about 75 and 150 nm. As stated before, said partially synthetic or wholly synthetic vesicle-forming lipid is preferably selected from the group consisting of DSPC, DPPC, HSPC and HEPC.

A microvesicle according to the present invention comprises a water-soluble corticosteroid. The term "water-soluble" is defined herein as having a solubility at a temperature of 25°C of at least 10 g/l water or water buffered at neutral pH.

Water soluble corticosteroids which can be advantageously used in accordance with the present invention are alkali metal and ammonium salts prepared from corticosteroids, having a free hydroxyl group, and organic acids, such as (C₂ - C₁₂) aliphatic, saturated and unsaturated dicarbonic acids, and inorganic acids, such as phosphoric acid and sulphuric acid. Also acid addition salts of corticosteroids can advantageously be encapsulated in the vesicles, preferably liposomes, more preferably long-circulating PEG-liposomes. If more than one group in the corticosteroid molecule is available for salt formation, mono- as well as di-salts may be useful. As alkaline metal salts the potassium and sodium salts are preferred. Also other, positively or negatively charged, derivatives of corticosteroids can be used. Specific examples of water soluble corticosteroids are betamethasone sodium phosphate, desonide sodium phosphate, dexamethasone sodium phosphate, hydrocortisone sodium phosphate, hydrocortisone sodium succinate, methylprednisolone disodium phosphate, methylprednisolone sodium succinate, prednisolone sodium phosphate, prednisolone sodium succinate, prednisolamate hydrochloride, prednisone disodium phosphate, prednisone sodium succinate, triamcinolone acetonide disodium phosphate and triamcinolone acetonide disodium phosphate.

Of these corticosteroids, prednisolone disodium phosphate, prednisolone sodium succinate, methylprednisolone disodium phosphate, methylprednisolone sodium succinate, dexamethasone disodium phosphate and betamethasone disodium phosphate are preferred.

As said, the microvesicles used in accordance with the present invention may be prepared according to methods used in the preparation of conventional liposomes and PEG-liposomes, as disclosed in or WO 02/45688 and WO 03/105805. Passive loading of the active ingredients into the liposomes by dissolving the corticosteroids in the aqueous phase is sufficient in order to reach an encapsulation as high as possible, but other methods can also be used. The lipid components used in forming the liposomes may be selected from a variety of vesicle-forming lipids, such as phospholipids, sphingolipids and sterols. Substitution (complete or partial) of these basic components by *e*.*g*. sphingomyelines and ergosterol appeared to be possible. For effective encapsulation of the, preferably water-soluble, corticosteroids in the microvesicles, thereby avoiding leakage of the drug from the microvesicles, especially phospholipid components having saturated, rigidifying acyl chains have appeared to be useful. The beneficial effects observed after one single injection of the water soluble corticosteroid containing PEG liposomes according to the invention are very favourable.

In addition, a composition used in accordance with the present invention may comprise one or more additional anti-atherosclerotic components. A non-limiting example of a preferred anti-atherosclerotic component is a statin.

In an additional aspect, the present invention also relates to novel pharmaceutical compositions. For instance, the invention relates to a pharmaceutical composition comprising a long-circulating microvesicle according to the invention, a corticosteroid contained therein and at least one anti-atherosclerotic compound, preferably a statin. Said pharmaceutical composition preferably comprises a pharmaceutically acceptable diluent, carrier or excipient.

According to the present invention, compositions comprising a corticosteroid in water soluble form, encapsulated in particular types of long-circulating microvesicles, are particularly suitable for counteracting and/or at least in part preventing atherosclerosis and/or cardiovascular disease. Further provided is therefore a method for treating a subject suffering from, or at risk of suffering from, cardiovascular disease, comprising administering to said subject a therapeutically effective amount of long-circulating microvesicles comprising a sterol, partially synthetic or wholly synthetic vesicle-forming phospholipids, and a corticosteroid in water soluble form, which microvesicles have a mean particle diameter size range of between about 75 and 150 nm and which microvesicles are non-charged or negatively charged at physiological conditions. As explained before, said microvesicles are preferably selected from the group consisting of liposomes, nanocapsules and polymeric micelles. In one preferred embodiment said microvesicle comprises at most 10 mole percent of negatively charged vesicle-forming phospholipids in order to increase the half-life of said microvesicle.

The invention is further explained in the following examples. These examples do not limit the scope of the invention, but merely serve to clarify the invention.

### Examples

### Example 1

### A novel nanomedicine-based anti-inflammatory treatment for advanced atherosclerotic lesions monitored by multimodality imaging

In this study we show the applicability of long circulating liposomes for efficient drug delivery to atherosclerotic plaques. Importantly, we were able to monitor their delivery by MRI. ¹⁸FDG-PET/CT was used to monitor therapeutic responses of the liposome-encapsulated glucocorticoids which revealed a significant and unprecedented reduction of inflammation of the atherosclerotic plaque was observed after a single injection of this agent.

In the present study we established the *in vivo* efficacy of liposome-encapsulated glucocorticoids in a rabbit model of atherosclerosis by monitoring their effects by ¹⁸F-FDG PET/CT while tracking liposome delivery by MRI using clinical scanners.

### Materials and Methods

### Animal protocol

Seventeen male New Zealand White (NZW) rabbits (mean age 7 months; mean weight 3.5 ± 0.2 kg; Covance) were included in this study. Aortic atherosclerotic plaques were induced in 15 NZW rabbits, through a well established model¹⁸, by a combination of 7 months of high cholesterol diet (4.7% palm oil and 0.3% cholesterol-enriched diet; Research Diet Inc.) and a repeated balloon injury of the aorta (two weeks and six weeks after starting the high cholesterol diet). Aortic injury was performed from the aortic arch to the iliac bifurcation with a 4F Fogarty embolectomy catheter introduced through the femoral artery. All procedures were performed under general anesthesia by an intramuscular injection of Ketamine (20 mg/kg; Fort Dodge Animal Health), Xylazine (10 mg/kg; Bayer Corp.) and Acepromazine (5 mg/kg: Boehringer Ingelheim). Two non-injured rabbits, fed a normal chow diet, were used as non-atherosclerotic controls. Plaque biology of induced atherosclerotic lesions of the abdominal aorta of rabbits closely resembles atherosclerotic lesions of humans; the diameter is approximately the size of a human coronary artery. All experiments were approved by the Mount Sinai School of Medicine Institute Animal Care and Use Committee.

### Glucocorticoids

Long-circulating paramagnetic liposomes containing glucocorticoids were prepared following modified procedures described previously (WO 03/105805, WO 02/45688 and (Mulder et al, Bioconjugate Chemistry 2004)). In brief, 51.5 % 1,2-dipalmitoyl-sn-Glycero-3-Phosphocholine (DPPC); 33.3 % cholesterol; 5.0 % 1,2-distearoyl-sn-glycero-3-phosphoethanolamine-N-[methoxy(polyethylene glycol)-2000] (PEG-DSPE); 10 % Gd-DTPA-bis(stearylamide) (Gd-DTPA-BSA); 0.2 % 1,2-dipalmitoyl-sn-glycero-3-phosphoethanolamine-N-(Lissamine Rhodamine B Sulfonyl) (Rhodamine-PE) were dissolved in chloroform:methanol (2:1 vol/vol) in a roundbottom flask. A lipid film was made under reduced pressure on a rotary evaporator and dried under a stream of nitrogen. Liposomes were formed by addition of an aqueous solution of 100 mg/ml prednisolone phosphate disodium salt (Bufa, Uitgeest, The Netherlands). A water-soluble phosphate derivative of prednisolone was used to ensure stable encapsulation in the liposomes. Liposome size was reduced by multiple extrusion steps through polycarbonate membranes (Nuclepore, Pleasanton, Calif., U.S.) with a final pore size of 100 nm. Mean particle size of the liposomes was determined to be 120 nm by dynamic light scattering. Phospholipid content was determined with a phosphate assay, performed on the organic phase after extraction of lipids with chloroform, according to Rouser. Unencapsulated GC were removed by dialysis in a Slide-A-Lyzer cassette with a molecular weight cut-off of 10 kDa at 4 °C with repeated changes of buffer. The aqueous phase after extraction was used for determining the glucocorticoid phosphate content by high performance liquid chromatography as described previously. The type of column was RP18 (5 µm) (Merck) and the mobile phase consisted of acetonitril and water (1:3 v/v), pH 2. The eluent was monitored with an ultraviolet detector set at 254 nm. The detection limit for the high performance liquid chromatography setup was 20 ng/ml. The final liposomal preparation contained about 5 mg GC/ml and 65 µmol phospholipid/ml.

### MRI

MRI was used to monitor delivery of liposome-encapsulated glucocorticoids into atherosclerotic plaques. Rabbits were sedated with Ketamine/Xylazine/Acepromazine (as above) and imaged supine in a 1.5-Tesla MRI clinical system (Siemens, Sonata, Germany) using high-resolution MR imaging (i.e., pre- and post-contrast, T1-weighted, TR 800 ms, TE 5.60 ms, FOV:120 x 120 mm, 3.00 tck/1.50 sp, 256 x 256/4.00 NEX). After a pre-treatment scan was performed, liposome-encapsulated glucocorticoids were administered under MRI guidance. MR images were acquired pre-injection, immediately after injection and 2, 7, 14 and 21 days post-injection of the liposomes.

### PET/CT

PET/CT was used to evaluate the therapeutic efficacy of liposome-encapsulated glucocorticoids. PET/CT scanning was performed on a GE advance LS 16 slice PET/CT scanner. This system has PET and CT components mounted back-to-back, and is mechanically calibrated so that alignment between the two parts is within 2 mm in the transaxial field of view. Rabbits were fasted for 4 hours prior to FDG injection, water ad libitum. They were scanned pre-injection and 2, 7, 14 and 21 days post-injection of the treatment.

Rabbits were kept still during imaging under general anesthesia and secured with a Velcro blanket. They were injected with 1 - 2 mCi/Kg ¹⁸F-FDG administered over 20 seconds via the marginal ear vein. Imaging started 180 minutes after ¹⁸F-FDG injection, which is the optimal time point according to previous studies¹⁹. PET imaging covered the region from the superior mesenteric artery to the iliac bifurcation. The bladder was emptied prior to image acquisition to reduce reconstruction artifacts of ¹⁸F-FDG in the urine and to give a clear view of the distal aorta. Images were acquired in full 3D mode; FOV 15.5 cm per bed; single bed coverage; 10 minutes per bed; FORE - IT reconstruction technique, 30 cm FOV, giving reconstructed slice thickness of 4.25 mm. PET images were calibrated to the injected dose of ¹⁸F-FDG.

### Experimental set-up

Rabbits were randomly assigned to receive a single injection of liposome-encapsulated glucocorticoids or free circulating glucocorticoids. The drugs were administered to the rabbits through the marginal ear vein at a dose of 15 mg/kg. Schematic 1 displays the different scanning and histology time points. 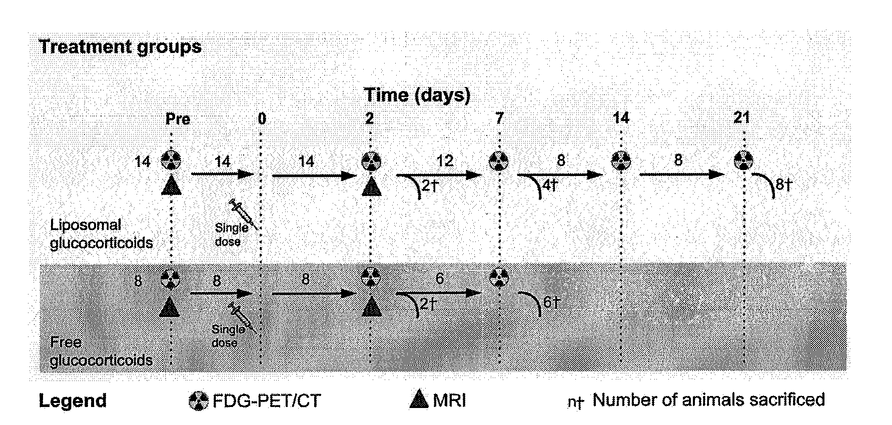 *Schematic 1* On the left a schematic representation of the liposomal nanomedicine is depicted. The liposomes are additionally labeled with paramagnetic and fluorescent lipids to allow MRI and fluorescence microscopy, respectively. On the right an overview of the experimental setup is given.

### Histology

Rabbits were sacrificed within 24 hours following last PET/CT acquisition by an intravenous injection of 120 mg/kg of sodium pentobarbital (Sleepaway; Fort Dodge Animal Health) at time points according to schematic 1. A bolus of heparin was injected prior to sacrifice to prevent clot formation. Aortas were excised, fixed for 24 hours in 4% paraformaldehyde and embedded in paraffin. Five-µm-thick slices were sectioned in the same direction as PET/CT slices and stained with Masson's trichrome. Macrophages were detected on adjacent slices by immunohistochemistry. Arterial sections were incubated with 0.3 % hydrogen peroxide to block endogenous peroxidase, then with a monoclonal mouse antibody to RAM-11, a marker of rabbit macrophage cytoplasm (dilution 1:200, Dako). Biotinylated polyclonal anti-mouse secondary antibody and peroxidase-conjugated streptavidin were applied for 30 minutes each with the use of the ABC Kit (Dako). Peroxidase activity was visualized by diaminobenzidine to yield brown cytoplasmic reaction products. Sections were counterstained with hematoxylin.

### Fluorescence microscopy

Immunofluorescence was performed on 8-µm-thick aortic sections. Confocal imaging was performed using a Zeiss LSM 510 META microscope (Carl Zeiss AG, Oberkochen, Germany) in an inverted configuration. Pinhole settings were adjusted for equal optical sections. Data were captured and analyzed using Zeiss LSM 510 Meta and Image Browser software (Carl Zeiss AG). PhotoShop CS 2 (Adobe Systems Inc., San Jose, Calif., U.S.) was used for post processing of images.

### Data analysis

PET/CT images were analyzed on a Xeleris workstation. SUV (Standard Uptake Value) is a measure to assess the amount of glucose uptake. SUV was calculated by dividing the tissue concentration (kBq/mL) by the injected dose per gram of body weight (kBq/g). The mean SUV was obtained from transaxial images of the abdominal aorta starting from the superior mesenteric artery down to the iliac bifurcation by registering a region of interest over the aorta.

For histology, RAM-11 macrophage positive areas were measured using ImageJ software.

### Statistical analysis

Data are presented as the mean ± SD. Statistical analysis was performed using paired t-test for comparisons within groups. Statistical significance was established at P < 0.05.

### Results

### Delivery and localization

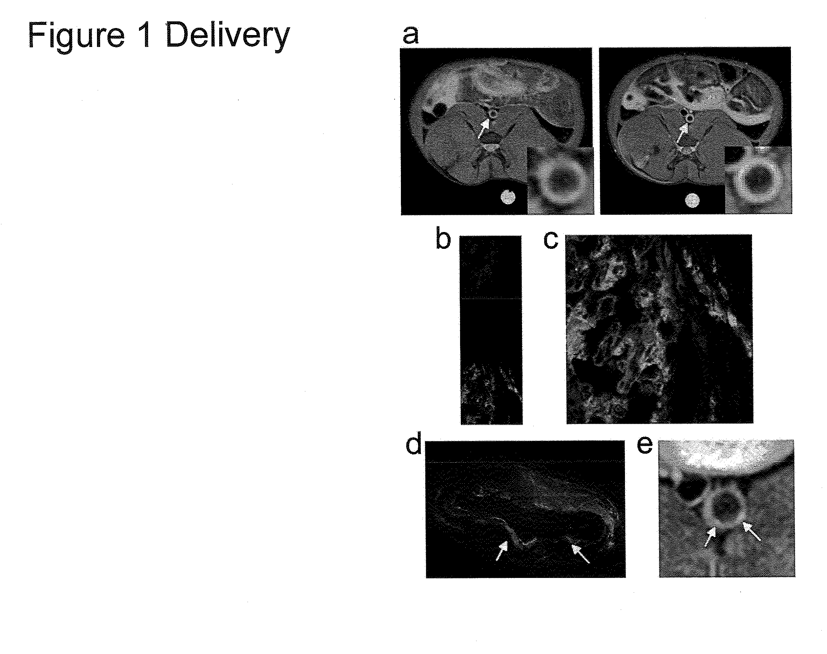

MRI of the abdominal aorta of atherosclerotic NZW rabbits was performed to monitor the delivery of the gadolinium-labeled liposomal corticosteroids after intravenous administration. Figure 1a shows an MR image of a rabbit aortic wall pre- and 2 days post-injection of the liposomes. Note the increased signal intensity on the post-injection scan due to the gadolinium label incorporated in the liposomes. MRI showed that the compound was localized in the aorta by showing heterogeneous signal enhancement over the entire abdominal aortic vessel wall; implying that liposomes had extravasated out of the circulation into the vessel wall.

To confirm signal enhancement on MRI occurred due to uptake of liposome-encapsulated glucocorticoids, fluorescence microscopy images were acquired of corresponding aortic sections. Figure 1b and c show that liposomes were mainly found to be associated with macrophages. Although in every section we found liposomes throughout the entire aortic plaque, the quantity of liposome accumulation was heterogeneously distributed. To investigate this we used reconstruction of an entire aortic section by merging the 10X fluorescent pictures (Figure 1d). The corresponding MRI image is shown in Figure 1e. The regions that contained a high proportion of rhodamine-labeled liposomes corresponded with hyperintense areas in the aortic wall (arrows Figure ld and e).

### Therapeutic effects of liposome-encapsulated glucocorticoids

To validate the therapeutic efficacy of liposome-encapsulated glucocorticoids, 17 NZW rabbits were scanned by ¹⁸FDG-PET/CT. These scans provide information about the level of inflammation present. The scans allow the quantification of the standard uptake value (SUV) of ¹⁸F-FDG in the abdominal aorta. The SUV is the decay-corrected tissue concentration of FDG (in kBq/g), corrected for injected FDG dose and body weight (in kBq/g), and is a well-recognized method for quantification of FDG PET data. Prior to start of treatment, the aortas of six atherosclerotic rabbits and two non-atherosclerotic rabbits were measured to establish mean SUV levels of atherosclerotic and non-atherosclerotic rabbits.

These measurements resulted in SUV levels of 0.65 ± 0.03 for atherosclerotic rabbits, comparable to SUV values in other studies²⁰, and 0.23 ± 0.03 for non-atherosclerotic rabbits.

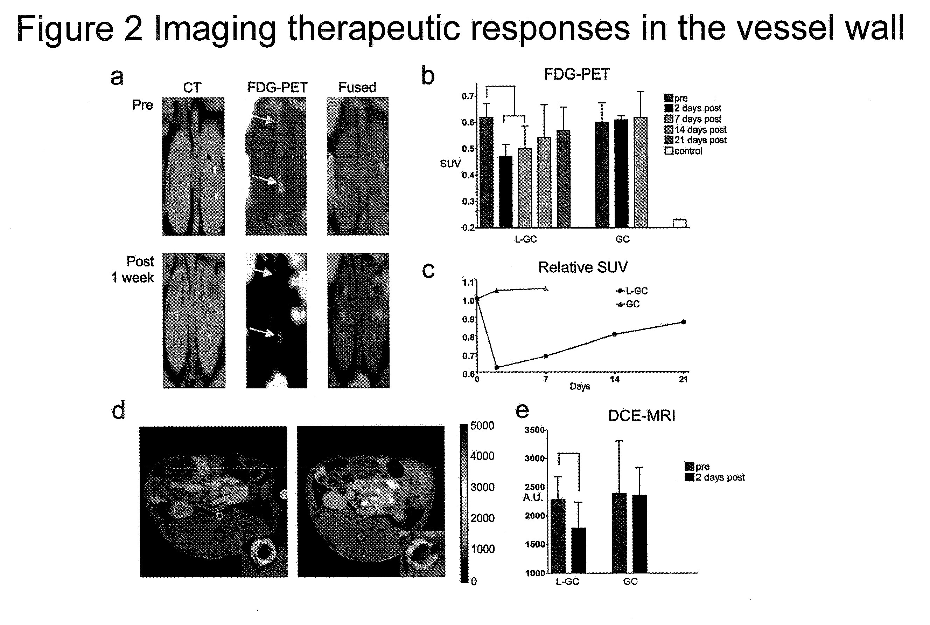

In Figure 2a, coronal CT and ¹⁸F-FDG-PET images of the aorta of an atherosclerotic rabbit are shown. Hotspots of ¹⁸F-FDG uptake were clearly visible throughout the aorta before treatment with liposome-encapsulated glucocorticoids (top), while a reduced FDG uptake was observed one week after treatment (bottom), demonstrating the effectiveness of liposome-encapsulated glucocorticoids. The mean SUV of the different groups, *i*.*e*. liposome-encapsulated glucocorticoid treated, free circulating glucocorticoid treated, and healthy animals at base level are shown in Figure 2b. One week post-treatment, rabbits injected with liposome-encapsulated glucocorticoids showed a significant SUV reduction (p = 0.006), compared to no SUV reduction in rabbits treated with free circulating glucocorticoids (p = 0.40). At 14 days post-treatment, a moderate, but not significant decrease was monitored, while at 21 days the mean SUV was back to base level. For animals that were treated with free circulating glucocorticoids no significant SUV differences were found between pre, and two and seven days post treatment.

The relative changes in SUV were determined by [(post-healthy/ (pre-healthy)] and are displayed in Figure 2c as an extraction of the previous graph. We observed that the relative SUV decreased 40% for rabbits treated with liposome-encapsulated corticosteroids compared to rabbits treated with free circulating corticosteroids. In addition, dynamic contrast enhanced MRI (DCE-MRI) was performed to quantify changes in plaque permeability. From DCE-MRI so-called area under the curve (AUC) maps can be generated (Figure 2d). To this aim, rabbits underwent DCE-MRI before and two days after the administration of the liposomes. A significant reduction of the AUC was found for the liposome group only, not the animals administered with free glucocorticoids (Figure 2e).

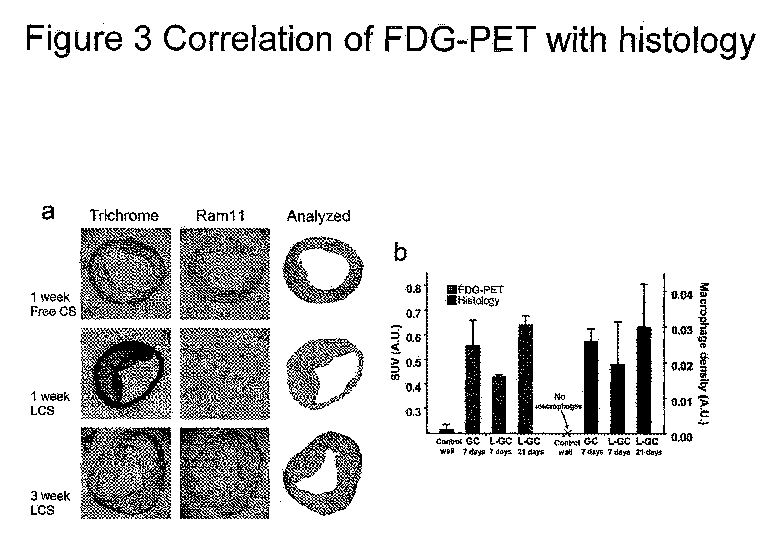

After the PET scans, aortas were excised and sectioned in segments corresponding to reconstructed PET/CT axial slices. Figure 3a shows images of Masson's trichrome and RAM-11 stained sections with different treatments/time points. Quantitative immunohistochemistry measurements of macrophages were performed to validate PET findings. With that objective, two animals from each group (healthy, 1 week LCS, 1 week free CS, 3 week LCS) were sacrificed and macrophage density was quantified (Figure 3a). A correlation between SUV and macrophage density in corresponding sections was observed (Figure 3b). Macrophage density in rabbits treated with liposome-encapsulated glucocorticoids was lower than in rabbits treated with free circulating glucocorticoids corresponding to findings from PET/CT scans. No macrophages were detected by immunohistochemistry in the aortic wall of healthy rabbits. Three weeks after treatment with liposome-encapsulated glucocorticoids macrophage density was increased, similar to findings of the three-week PET/CT scan.

### Discussion

In this study we have shown (I) the potential applicability of long-circulating liposomes as a drug carrier system for efficient delivery to atherosclerotic plaques. In addition we were able to (II) monitor the delivery of these liposomes by MRI, which was confirmed using immunofluorescence techniques at cellular level. In our study glucocorticoids were encapsulated into long-circulating liposomes. We demonstrated that (III) FDG-PET/CT can be used to monitor therapeutic responses of liposome-encapsulated glucocorticoids. In addition, we found that (IV) a single injection of the liposome-encapsulated glucocorticoids showed a therapeutic effect within two days, lasting up to two weeks, before returning to baseline after three weeks. Most clinically used anti-inflammatory agents need to be administrated for weeks or even months before they are effective. To the best of our knowledge a comparable effective method to inhibit inflammation in atherosclerosis has not been reported before.

*In vivo* and *ex vivo* imaging showed that long-circulating liposomes are highly suitable as a carrier vehicle for drug delivery to atherosclerotic plaques. The therapeutic efficacy of the administration of liposome-encapsulated glucocorticoids is dramatically better than free circulating glucocorticoids, for which we did not observe significant changes of the inflammatory state of the atherosclerotic lesions. High quantities of liposomes were found in the atherosclerotic plaque. Without wishing to be bound to theory, the accumulation can be attributed to extravasation of the liposomes from the circulation into atherosclerotic areas rich in neovessels, which are characterized by a permeable endothelium. Due to the long- circulating properties of the liposomes a high proportion of the injected dose ends up in the plaques. As well as passive targeting of liposome-encapsulated glucocorticoids to sites with enhanced capillary permeability, active targeting of *e*.*g*. angiogenic endothelial cells may be accomplished by conjugation of targeting ligands to the liposomal surface.

### Brief description of the drawings

**Figure 1.** (a) In vivo MRI of the abdominal aorta before (left) and two days after (right) the administration of liposomes. Pronounced uptake was observed throughout the atherosclerotic lesion. (b) Confocal laser scanning microscopy (CSLM) of liposomes (red), cell nuclei (blue), and macrophages. (c) A high degree of co-localization of liposomes with macrophages was observed. (d) Although liposomes were found throughout the entire lesion areas, a vessel wall reconstruction of multiple CLSM images revealed heterogeneous accumulation of lipsosomes. (e) The corresponding MRI slice of the histological section depicted in (e) revealed a similar heterogeneous distribution.
**Figure 2.** (a) A representative coronal CT, FDG-PET, and fused slice throughout the abdominal aorta of an atherosclerotic rabbit before and 1 week post administration of liposomal glucocorticoids (b) The mean SUV for the different time points pre and post injection of liposomal glucocorticoids and free glucocorticoids are given. (c) Relative changes in SUV for animals treated with liposomal and free glucocorticoids. (d) Overlays on anatomical images of area under the curve (AUC) maps obtained with DCE MRI before (left) and two days post (right) treatment with liposomal glucocorticoids. (e) The mean AUC values of the aortic wall of animals treated with liposomal (left) or free (right) glucocorticoids.
**Figure 3.** (a) Representative histological slices of aortic section stained with trichrome and stained for macrophages with Ram11. The macrophage density was quantified. (b) The mean SUV (left) for the rabbits that underwent histological quantification of macrophage density (right).

### References

1. Ross, R. Atherosclerosis--an inflammatory disease. N Engl J Med 340, 115-26 (1999).
2. Strong, J.P. et al. Prevalence and extent of atherosclerosis in adolescents and young adults: implications for prevention from the Pathobiological Determinants of Atherosclerosis in Youth Study. Jama 281, 727-35 (1999).
3. Poon, M. et al. Dexamethasone inhibits macrophage accumulation after balloon arterial injury in cholesterol fed rabbits. Atherosclerosis 155, 371-80 (2001).
4. Ribichini, F. et al. Effects of oral prednisone after stenting in a rabbit model of established atherosclerosis. J Am Coll Cardiol 50, 176-85 (2007).
5. Goulding, N.J. & Guyre, P.M. Glucocorticoids, lipocortins and the immune response. Curr Opin Immunol 5, 108-13 (1993).
6. Amsterdam, A., Tajima, K. & Sasson, R. Cell-specific regulation of apoptosis by glucocorticoids: implication to their anti-inflammatory action. Biochem Pharmacol 64, 843-50 (2002).
7. Maderna, P. & Godson, C. Phagocytosis of apoptotic cells and the resolution of inflammation. Biochim Biophys Acta 1639, 141-51 (2003).
8. Schiffelers, R.M., Banciu, M., Metselaar, J.M. & Storm, G. Therapeutic application of long-circulating liposomal glucocorticoids in auto-immune diseases and cancer. J Liposome Res 16, 185-94 (2006).
9. Hrynyk, R., Storm, G., Metselaar, B. & Langner, M. Pharmacokinetics of liposomes designed to carry glucocorticoids. Pol J Pharmacol 55, 1063-70 (2003).
10. Asgeirsdottir, S.A. et al. Site-specific inhibition of glomerulonephritis progression by targeted delivery of dexamethasone to glomerular endothelium. Mol Pharmacol 72, 121-31 (2007).
11. Torchilin, V.P. Recent advances with liposomes as pharmaceutical carriers. Nat Rev Drug Discov 4, 145-60 (2005).
12. Banciu, M., Schiffelers, R.M., Fens, M.H., Metselaar, J.M. & Storm, G. Anti-angiogenic effects of liposomal prednisolone phosphate on B16 melanoma in mice. J Control Release 113, 1-8 (2006).
13. Schmidt, J. et al. Drug targeting by long-circulating liposomal glucocorticosteroids increases therapeutic efficacy in a model of multiple sclerosis. Brain 126, 1895-904 (2003).
14. Metselaar, J.M., Wauben, M.H., Wagenaar-Hilbers, J.P., Boerman, O.C. & Storm, G. Complete remission of experimental arthritis by joint targeting of glucocorticoids with long-circulating liposomes. Arthritis Rheum 48, 2059-66 (2003).
15. Iyer, A.K., Khaled, G., Fang, J. & Maeda, H. Exploiting the enhanced permeability and retention effect for tumor targeting. Drug Discov Today 11, 812-8 (2006).
16. Zhang, L. et al. Nanoparticles in Medicine: Therapeutic Applications and Developments. Clin Pharmacol Ther (2007).
17. Zhang, Z. et al. Non-invasive imaging of atherosclerotic plaque macrophage in a rabbit model with F-18 FDG PET: a histopathological correlation. BMC Nucl Med 6, 3 (2006).
18. Hyafil, F. et al. Noninvasive detection of macrophages using a nanoparticulate contrast agent for computed tomography. Nat Med 13, 636-41 (2007).
19. Rudd, J.H. et al. Imaging atherosclerotic plaque inflammation with [18F]-fluorodeoxyglucose positron emission tomography. Circulation 105, 2708-11 (2002).
20. Ogawa, M. et al. (18)F-FDG accumulation in atherosclerotic plaques: immunohistochemical and PET imaging study. J Nucl Med 45, 1245-50 (2004).
21. Amirbekian, V. et al. Detecting and assessing macrophages in vivo to evaluate atherosclerosis noninvasively using molecular MRI. Proc Natl Acad Sci U S A 104, 961-6 (2007).
22. Mulder, W.J. et al. MR molecular imaging and fluorescence microscopy for identification of activated tumor endothelium using a bimodal lipidic nanoparticle. Faseb J 19, 2008-10 (2005).
23. Choudhury, R.P., Fuster, V. & Fayad, Z.A. Molecular, cellular and functional imaging of atherothrombosis. Nat Rev Drug Discov 3, 913-25 (2004).
24. Ogawa, M. et al. Application of 18F-FDG PET for monitoring the therapeutic effect of antiinflammatory drugs on stabilization of vulnerable atherosclerotic plaques. J Nucl Med 47, 1845-50 (2006).
25. Tahara, N. et al. Simvastatin attenuates plaque inflammation: evaluation by fluorodeoxyglucose positron emission tomography. J Am Coll Cardiol 48, 1825-31 (2006).
26. Rudd, J.H. et al. (18)Fluorodeoxyglucose positron emission tomography imaging of atherosclerotic plaque inflammation is highly reproducible: implications for atherosclerosis therapy trials. J Am Coll Cardiol 50, 892-6 (2007).
27. Winter, P.M. et al. Endothelial alpha(v)beta3 integrin-targeted fumagillin nanoparticles inhibit angiogenesis in atherosclerosis. Arterioscler Thromb Vasc Biol 26, 2103-9 (2006).
WO 02/45688
WO 03/105805

Chono, S; Tauchi, Y; Morimoto, K. Aortic drug delivery of dexamethasone palmitate incorporated into lipid microspheres and its antiatherosclerotic effect in atherogenic mice. J.Drug Target. 2005a. Aug;13(7): 407-414

Chono, S; Tauchi, Y; Deguchi, Y; Morimoto, K. Efficient drug delivery to atherosclerotic lesions and the antiatherosclerotic effect by dexamethasone incorporated into liposomes in atherogenic mice. J Drug Target. 2005 May;13(4):267-76

Derendorf H, Rohdewald P, Hochhaus G, Möllmann H. HPLC determination of glucocorticoid alcohols, their phosphates and hydrocortisone in aqueous solutions and biological fluids. J Pharm Biomed Anal. 1986; 4(2): 197-206.

Goulding NJ, Guyre PM. Glucocorticoids, lipocortins and the immune response. Curr Opin Immunol. 1993 Feb;5(1):108-13

Maderna P, Godson C. Phagocytosis of apoptotic cells and the resolution of inflammation. Biochim Biophys Acta. 2003 Nov 20;1639(3):141-51

Mulder WJ, Strijkers GJ, Griffioen AW, van Bloois L, Molema G, Storm G, Koning GA, Nicolay K. A liposomal system for contrast-enhanced magnetic resonance imaging of molecular targets. Bioconjug Chem. 2004 Jul-Aug;15(4):799-806. PMID: 15264867 [PubMed - indexed for MEDLINE]

Poon M, Gertz SD, Fallon JT, Wiegman P, Berman JW, Sarembock IJ, Taubman MB. Dexamethasone inhibits macrophage accumulation after balloon arterial injury in cholesterol fed rabbits. Atherosclerosis. 2001 Apr;155(2):371-80.

Ribichini F, Joner M, Ferrero V, Finn AV, Crimins J, Nakazawa G, Acampado E, Kolodgie FD, Vassanelli C, Virmani R. Effects of oral prednisone after stenting in a rabbit model of established atherosclerosis. J Am Coll Cardiol. 2007 Jul 10;50(2):176-85. Epub 2007 Jun 22.

Rouser G, Fkeischer S, Yamamoto A. Two-dimensional thin layer chromatographic separation of polar lipids and determination of phospholipids by phosphorus analysis of spots. Lipids 1970;5:494-6. 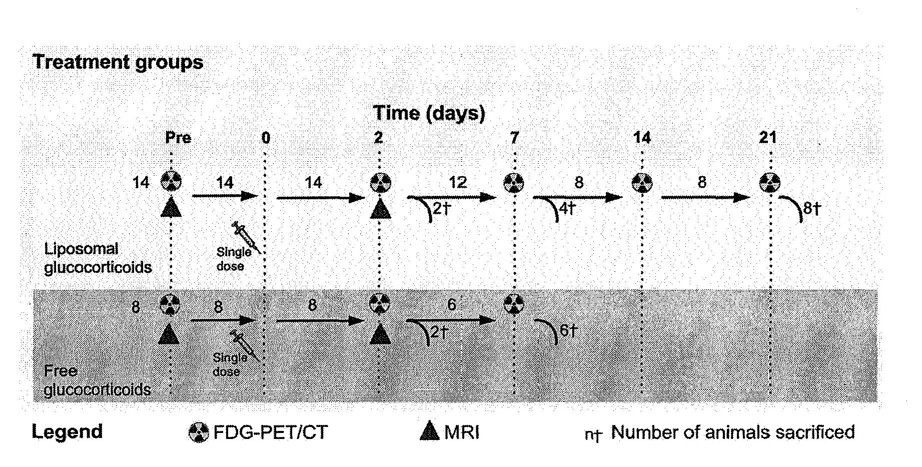 Schematic. On the left a schematic representation of the liposomal nanomedicine is depicted. The liposomes are additionally labeled with paramagnetic and fluorescent lipids to allow MRI and fluorescence microscopy, respectively. On the right an overview of the experimental setup is given.

## Claims

1. Use of a long-circulating microvesicle comprising a sterol, partially synthetic or wholly synthetic vesicle-forming phospholipids, and a corticosteroid in water soluble form, which microvesicle has a mean particle diameter size range of between about 75 and 150 nm and which microvesicle is non-charged or negatively charged at physiological conditions, for the preparation of a medicament for the treatment of atherosclerosis and/or cardiovascular disease.

2. Use according to claim 1, wherein said microvesicle is selected from the group consisting of liposomes, nanocapsules and polymeric micelles.

3. Use according to claim 1 or 2, wherein said microvesicle comprises at most 10 mole percent of negatively charged vesicle-forming phospholipids.

4. Use according to any one of claims 1-3, wherein said microvesicle further comprises polyethylene glycol.

5. Use according to any one of claims 1-4, wherein said sterol comprises cholesterol.

6. Use according to any one of claims 1-5, wherein said microvesicle is a liposome comprising 0-50 mol% of cholesterol, 50-90 mol% of non-charged partially synthetic or wholly synthetic vesicle-forming lipids, 0-20 mol% of amphipatic vesicle-forming lipids coupled to polyethylene glycol, and 0-20 mol% of a negatively charged vesicle-forming lipid.

7. Use according to any one of claims 1-6, wherein said partially synthetic or wholly synthetic vesicle-forming phospholipids contain saturated alkyl chains.

8. Use according to any one of claims 1-7, wherein said microvesicle has a circulation half-life of at least 3 hours, preferably at least 6 hours.

9. A method for treating a subject suffering from, or at risk of suffering from, atherosclerosis and/or cardiovascular disease, comprising administering to said subject a therapeutically effective amount of long-circulating microvesicles comprising a sterol, partially synthetic or wholly synthetic vesicle-forming phospholipids, and a corticosteroid in water soluble form, which microvesicles have a mean particle diameter size range of between about 75 and 150 nm and which microvesicles are non-charged or negatively charged at physiological conditions.

10. A method according to claim 9, wherein said microvesicles are selected from the group consisting of liposomes, nanocapsules and polymeric micelles.

11. A pharmaceutical composition comprising:
- a long-circulating microvesicle comprising a sterol, partially synthetic or wholly synthetic vesicle-forming phospholipids, and a corticosteroid in water soluble form, which microvesicle has a mean particle diameter size range of between about 75 and 150 nm and which microvesicle is non-charged or negatively charged at physiological conditions; and
- at least one other anti-atherosclerotic compound.
